Europäisches Patentamt

European Patent Office

Office européen des brevets

(19)

(11) Publication number: 0 375 068
A1

# EUROPEAN PATENT APPLICATION

(21) Application number: 89203250.9

(22) Date of filing: 18.12.89

(51) Int. Cl.⁵: A61K 33/24, A61K 31/65,
A61K 31/29, //(A61K33/24,
31:65,31:29)

(30) Priority: 23.12.88 EP 88203006

(43) Date of publication of application:
27.06.90 Bulletin 90/26

(84) Designated Contracting States:
AT BE CH DE ES FR GB GR IT LI LU NL SE

(71) Applicant: GIST-BROCADES N.V.
Wateringseweg 1 P.O. Box 1
NL-2600 MA Delft(NL)

(72) Inventor: Castelijns, Lodewijk Franciscus
Pancratius
Kerkstraat 29
NL-5529 AK Casteren(NL)

(74) Representative: Lavy, Uriel et al
Gist-Brocades NV Patents and Trademarks
Dept. Wateringseweg 1 P.O. Box 1
NL-2600 MA Delft(NL)

(54) Combining bismuth compounds and tetracyclines.

(57) The use of bismuth compounds and tetracyclines in admixture for the manufacture of oral preparations for the treatment of disorders of the upper gastrointestinal tract, and pharmaceutical preparations containing said ingredients in admixture. Preferred bismuth compounds are colloidal bismuth subcitrate and bismuth subsalicylate. A preferred tetracycline is tetracycline-HCl.

EP 0 375 068 A1

## Combining bismuth compounds and tetracyclines

This invention relates to compositions for the oral treatment of disorders of the upper gastrointestinal tract. Such disorders include, for example, peptic ulcer diseases such as gastric, duodenal and jejunal ulcers, oesophageal ulcers, and non-ulcerating gastrointestinal disorders such as oesophagitis, gastritis and duodenitis.

Among the factors which are known to be involved in causing or perpetuating disorders of the upper gastrointestinal tract, one that has had particular interest focused on it during the last decennium is the infective factor. In particular the spiral bacteria currently classified under the genus Campylobacter have been implicated, and more in particular Campylobacter pylori, formerly called C.pyloridis (C.S. Goodwin et al., J. Clin. Pathol. 1986, 39, 353-365).

Among the many preparations which are currently in use against one or more of the diseases of the upper gastrointestinal tract are those containing bismuth compounds, in particular colloidal bismuth subcitrate (CBS, formerly termed tripotassium dicitrato bismuthate) marketed by Gist-brocades under the trademark DE-NOL, and bismuth subsalicylate, marketed by Procter and Gamble under the trademark PEPTO-BISMOL. Patents directed to two solid preparations of CBS are GB 1478742 and EP 0075992. Beside being an established peptic ulcer healer, CBS is also known to reduce the relapse rate of healed ulcers (G.N.J. Tijtgat, Digestion 1987, 37 suppl. 2, 31-41). One explanation for these effects is the anti-bacterial activity of this and the other bismuth compounds, in particular against C.pylori. In vitro the mean $MIC_{50}$ of CBS against C.pylori as given in the literature varies from 8 to 16 $\mu g/ml$ (J.C. Dent and C.A.M. McNulty, Acta Endoscopica 1988, 18, 23-24; Y. Glupzynski et al., Europ. J. Epidemiol. 1988, 4, 154-157), which is not very impressive. Nevertheless, clinical studies in humans suffering from peptic ulcers have shown CBS to clear C.pylori in up to 50% of cases, which compares favourably with other antibacterial or antiulcer drugs (E.A.J. Rauws, M.L. Langenberg, P.J.G.M. Rietstra, H.C. Zanen, H.J. Houthoff and G.N.J. Tijtgat, Nederlandse Vereniging voor Gastroenterologie, Programma Voorjaarsvergadering 21-22 maart 1986, p. 37).

Many antibacterial drugs, such as the penicillins, the macrolides and the tetracyclines can inhibit or kill C.pylori in vitro. However, their effects in vivo are often disappointing, due to various reasons. For example, tetracycline HCl has been shown in vitro to have a mean $MIC_{50}$ of 0.08-0.25 $\mu g/ml$ against C.pylori (J.C. Dent and C.A.M. McNulty, above; T. Lambert et al., Antimicrob.

Agents & Chemotherap. 1986, 30, 510-511), while in vivo it cleared the bacterium from only 20% of peptic ulcer patients (E.A.J. Rauws, et al., above).

The administration of bismuth compounds "in conjunction" with antibiotics for the treatment of gastrointestinal disorders has been put forward in WO 8605981, and again in EP 0206625. Both mention the tetracyclines among the many antimicrobials which are stated to be suitable for such combinations. However, the only antimicrobial which is illustrated in the Examples of both is amoxycillin. WO 8605981 does not elaborate on what exactly is meant by "in conjunction", but EP 0206625 specifically warns on pages 8-10 against the simultaeous administration of a bismuth compound and an antimicrobial which is prone to interaction with it. In particular according to EP 0206625 "the presence of bismuth is known to adversely affect the efficacy of the tetracyclines". Hence EP 0206625 recommends "to administer those antimicrobials that are subject to adverse bismuth interactions by methods that minimize such interactions, i.e. by minimizing the simultaneous presence of antimicrobial and bismuth in the stomach". It is indeed well known that the tetracyclines tend to form chelates with bi- and trivalent metal ions. For this reason the literature contains numerous warnings against the concurrent oral administration of tetra cyclines and e.g. antacids or bismuth salicylate, which are said to inactivate the tetracyclines by reducing their absorption (Martindale's Extra Pharmacopoeia 1982, 1218). This has also been demonstrated with the concurrent administration of tetracycline with a soluble bismuth-citrate-peptide chelate (R.K. Mapp & T.J. McCarthy, S. Afr. J. Lab. & Clin. Med. 1976, 1829-1830). Thus from WO 8605981 and EP 0206625, combined with background art, it is clear that the separate administration of the bismuth and the tetracycline is intended, and that the goal is an enhanced antibacterial activity.

While tetracyclines, when administered orally, are generally intended to be absorbed, bismuth compounds nowadays are not. Their use, orally or per injection, for the systemic treatment of generalized infections such as syphilis is completely obsolete and they are now only used for local treatments such as that of gastrointestinal disorders. Absorbed bismuth is not entirely innocent, and massive doses of some of the older bismuth containing preparations have been known to cause reversible encephalopathies (G. Martin-Bouyer, Gastroenterol. Clin. Biol. 1978, 2, 349-356). The more modern bismuth preparations, such as CBS, are designed and dosed to guarantee a minute degree of absorption and - at least when used in the recommended way - they do not cause any toxicological disturbances (J.P. Bader, Digestion 1987, 37 suppl. 2, 53-59). Nevertheless an even further decrease of bismuth absorption from these preparations, provided such decrease does not involve a substantial loss of clinical efficacy, will be most welcome.

It has now been found that when bismuth compounds are given orally in combination with tetracyclines, the local anti-bacterial activity and the other local healing activities of the respective compounds used are retained, while their absorption into the bloodstream is substantially reduced. The combinations intended here are intimate mixtures.

The invention therefore concerns the use of bismuth compounds and tetracycline antibiotics for the manufacture of oral preparations for the treatment of disorders of the upper gastrointestinal tract, characterized in that the bismuth compound and the tetracycline antibiotic are used in admixture and not physically separated.

The invention also concerns pharmaceutical preparations for the oral treatment of disorders of the upper gastrointestinal tract, characterized in that they contain an intimate mixture without physical separation of a bismuth compound and a tetracycline antibiotic in amounts effective to ameliorate said disorders, optionally in combination with a pharmaceutically acceptable carrier.

Examples of suitable bismuth compounds are its aluminate, subcarbonate, citrate, subgallate, subnitrate, tartrate and any mixtures thereof.

A preferred bismuth compound is colloidal bismuth subcitrate. Another preferred bismuth compound is bismuth subsalicylate.

Examples of suitable tetracycline antibiotics are tetracycline, chlortetracycline, oxytetracycline and doxycycline.

A preferred tetracycline antibiotic is tetracycline HCl.

The doses of the bismuth compound and the tetracycline antibiotic used will depend on the particular ingredients used and on the medical indication concerned. Generally, a dosage range of from 10 mg to 10 grams of bismuth daily is feasible, combined with from 10 mg to 10 grams of a tetracycline antibiotic daily.

Preferably the amount of tetracycline in preparations according to the invention exceeds the amount of bismuth.

One dosage unit of a preparation according to the invention will contain 125-1000 mg of a tetracycline antibiotic and 27-216 mg of bismuth. A preferred preparation contains 500 mg of tetracycline HCl and 160 mg of colloidal bismuth subcitrate per dosage unit, preferably to be dosed 4 times per day.

**Claims**

1. The use of bismuth compounds and tetracycline antibiotics for the manufacture of oral preparations for the treatment of disorders of the upper gastrointestinal tract, characterized in that the bismuth compound and the tetracycline antibiotic are used in admixture and not physically separated.

2. Pharmaceutical preparations for the oral treatment of disorders of the upper gastrointestinal tract, characterized in that they contain an intimate mixture without physical separation of a bismuth compound and a tetracycline antibiotic in amounts effective to ameliorate said disorders.

3. Pharmaceutical preparations according to claim 2, characterized in that the bismuth compound is an aluminate, subcarbonate, citrate, subgallate, subnitrate, tartrate or any mixture thereof.

4. Pharmaceutical preparations according to claim 2, characterized in that the bismuth compound is colloidal bismuth subcitrate.

5. Pharmaceutical preparations according to claim 2, characterized in that the bismuth compound is bismuth subsalicylate.

6. Pharmaceutical preparations according to any of claims 2-5, characterized in that the tetracycline antibiotic is tetracycline HCl.

7. Pharmaceutical preparations according to any of claims 2-6, characterized in that the amount of the tetracycline in the preparation exceeds the amount of bismuth.

8. Pharmaceutical preparations according to any of claims 2-7, characterized in that they contain per dosage unit 27-216 mg of bismuth and 125-1000 mg of a tetracycline antibiotic.

9. Pharmaceutical preparations according to claim 8, characterized in that they contain per dosage unit 160 mg of colloidal bismuth subcitrate and 500 mg of tetracycline HCl.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 77, no. 21, 20th November 1972, page 29, abstract no. 135209k, Columbus, Ohio, US; J. VYHNALEK et al.: "Application of terapeutan in diarrhoeic diseases of calves and piglets", & VETERINARIA (PRAGUE) 1972, 14(1), 33-43<br>* Abstract *<br>--- | 1-9 | A 61 K 33/24<br>A 61 K 31/65<br>A 61 K 31/29 //<br>(A 61 K 33/24<br>A 61 K 31:65<br>A 61 K 31:29 ) |
| A | CHEMICAL ABSTRACTS, vol. 68, no. 19, 6th May 1968, page 8682, abstract no. 85855g, Columbus, Ohio, US; T. SCIORTINO et al.: "Tetracycline-bismuth incompability in pharmaceutical preparations", & BOLL. CHIM. FARM. 107(1), 47-53<br>* Abstract *<br>--- | 1-9 | |
| D,A | WO-A-8 605 981 (T.J. BORODY)<br>--- | 1-9 | |
| D,A | EP-A-0 206 625 (B.J. MARSHALL)<br>----- | 1-9 | TECHNICAL FIELDS SEARCHED (Int. Cl.5)<br><br>A 61 K |

The present search report has been drawn up for all claims

| Place of search | Date of completion of the search | Examiner |
|---|---|---|
| THE HAGUE | 13-03-1990 | BRINKMANN C. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
........................................................................................
& : member of the same patent family, corresponding
    document

EPO FORM 1503 03.82 (P0401)